# EUROPEAN PATENT SPECIFICATION

(11) **EP 2 194 979 B1**
(45) Date of publication and mention of the grant of the patent: **13.03.2013**
(21) Application number: 08807065.1
(22) Date of filing: 08.09.2008
(51) Int. Cl.: A61K 31/4015, A61P 25/00, A61P 25/06, A61P 25/24

(54) **TREATMENT OF CNS AND PAIN DISORDERS**
BEHANDLUNG VON ZNS-ERKRANKUNGEN UND SCHMERZEN
TRAITEMENT DE TROUBLES DU SYSTÈME NERVEUX CENTRAL ET DE LA DOULEUR

(30) Priority: 06.09.2007 US 851229
(43) Date of publication of application: 16.06.2010
(73) Proprietor: Xenova, Moscow 105082 (RU)
(72) Inventor: GRANIK, Vladimir G., 125565 Moscow (RU); PARSHIN, Valeryi A., 117574 Moscow (RU); SOROKINA, Irina K., 109382 Moscow (RU)
(74) Representative: Nevant, Marc
(86) International application number: PCT/IB2008/002378
(87) International publication number: WO 2009/031029

(56) References cited:
- RU-C1- 2 144 918

## Description

### FIELD OF THE INVENTION

The present invention relates to a method of curative and prophylactic treatment of a person suffering from a peripheral and central nervous system disorder, and to a corresponding means.

### BACKGROUND OF THE INVENTION

*Treatment* as used herein comprises curative treatment and prophylactic treatment.

*Curative* as used herein relates to the efficacy in treating ongoing episodes (e.g. depressive episodes in bipolar disorders).

*Prophylactic* as used herein relates to the prevention of the onset or recurrence or relapse of episodes (e.g. depressive or neuropathic pain episodes).

*Therapeutically effective amount* as used herein is the amount of a drug or pharmaceutical agent that elicits a desired biological or medical response of a tissue, system, animal or human sought by a researcher or clinician.

*Migraine* as used herein is a disorder characterized by recurrent attacks of headache that vary widely in intensity, frequency, and duration.

Headache is commonly unilateral and frequently associated with anorexia, nausea, vomiting, phonophobia, and/or photophobia. In some cases they are preceded by, or associated with, neurologic and mood disturbances. Migraine headache may last from about 4 hours to about 72 hours. The International Headache Society (1988) classifies migraine with aura (classical migraine) and migraine without aura (common migraine) as the two major types of migraine. Migraine with aura consists of a headache phase preceded by characteristic visual, sensory, speech, or motor symptoms. In the absence of such symptoms, the headache is called migraine without aura.

One-year prevalence figures are primarily dependent on age and sex (Ferrari M D, Migraine. The Lancet (1998) 351:1043-1051; Sheffield R E, Migraine prevalence: a literature review. Headache (1998) 38:595-601). Ten percent of the general population (6 % of males and 15 % of females) is suffering from migraine. Prevalence peaks:around 35 to 50 years of age in women and 25 to 35 years of age in men. Within the 10 to 19 years age group, there is a sharp increase in prevalence with age, with a peak around 14-16 years. Before puberty there is an equal prevalence between males and females. Among adults, the ratio of women to men is approximately 2.5:1. One-year prevalence rates for migraine without aura are 1.5 to 7 times higher than for migraine with aura.

Treatment of migraine can be divided into four types: general measures, abortive therapy, pain relief measures, and prophylactic treatment (Silberstein S D, Preventive treatment of migraine: an overview. Cephalalgia (1997) 17:67-72; Diamond S and Diamond M L, Contemporary diagnosis and management of headache and migraine (1998), 1st Ed., Handbooks in Health Care Co., Newtown, Pa., USA; Diener H C. et al. A practical guide to the management and prevention of migraine. Drugs (1998) 56(5):811-824).

General measures may be a regular sleep schedule, a regular meal schedule, dietary measures, etc. There are a variety of agents that can be used as abortive treatment, ranging from simple analgesics, such as acetyl salicylic acid, non-steroidal anti-inflammatory drugs (NSAIDs), ergotamine compounds and antiemetics, to recently developed serotonin (5-HT) agonists, such as triptan compounds.

Pain relief measures may include administration of NSAIDs or narcotic analgesics and rescue therapy. As for acute treatments there are a variety of medications that are used in the prophylaxis of migraine. Prophylactic treatment is usually given daily for months or years. It should be considered for patients who have two or more migraine attacks per month. β-Adrenoceptor blockers, primarily propranolol, have been recognized for their efficacy in migraine prevention. Equally efficacious is the antiepileptic drug divalproex sodium. Other compounds are tricyclic antidepressants (amitriptyline), calcium channel blockers (nifedipine, flunarizine, verapamil), NSAIDs (ketoprofen, naproxen), riboflavin (vitamin B2), and 5-HT antagonists.

Traditional theories of migraine pathogenesis include the vasogenic theory and the neurogenic theory. Neither of these theories completely explains all of the clinical phenomena observed during a migraine attack.

Current views on migraine pathophysiology take into account both neurological and vascular events in the initiation of an attack. Endogenous neurophysiologic events activate trigeminovascular fibers in the brain stem, with subsequent perivascular release of powerful vasoactive neuropeptides. In animal experiments these neuropeptides promote a neurogenic inflammation response, consisting of vasodilatation and dural plasma extravasation. Cortical spreading depression (CSD) has been described as slowly spreading waves of inhibition of cortical neurons associated with clinical symptoms of aura. Experimental CSD can activate the trigemino-vascular system in the brain stem, providing a possible link between aura and headache mechanisms.

There is some evidence for 5-HT acting as a neurotransmitter and humoral mediator in the neural and vascular components of the migraine headache. Migraine patients exhibit a systemic disturbance of the 5-HT metabolism.

It is theorized that persons prone to migraine have a reduced threshold for neuronal excitability, possibly due to diminished activity of the inhibitory neurotransmitter γ-aminobutyric acid (GABA). GABA normally reduces the cellular effects of the neurotransmitter serotonin (5-HT) and glutamate, both of which appear to be involved in migraine attacks.

Divalproex sodium is a stable co-ordination compound comprising sodium valproate and valproic acid in a 1:1 molar relationship. Disordered GABA metabolism has been reported in migraine patients and changes in cerebrospinal GABA levels were found during migraine episodes. Divalproex sodium is thought to elevate brain levels of γ-aminobutyric acid (GABA) by decreasing its degradation. The increased activity of GABAergic systems may influence the migraine generation directly or indirectly through a number of mechanisms. Potential indirect mechanisms include decreasing the firing rate of the serotonergic neurons in the dorsal raphe nucleus. The term "valproate" as used herein includes valproic acid and the derivatives such as valpromide, valproate pivoxil, magnesium valproate, divalproex sodium, sodium valproate and semi-sodium valproate. Valproate has been reported to raise endogenous brain levels of enkephalin, which plays a determinant role in analgesia.

It has been postulated that valproate decreases levels of excitatory amino acids in the brain, interfering with CSD (Mathew N T et al., Migraine prophylaxes with divalproex. Arch Neurol (1995) 52:281-286; Welch K M et al, The concept of migraine as a state of central neuronal hyperexcitability. Neurol Clin (1990) 8:817-8281).

The most common side effects reported with valproate are nausea, vomiting, indigestion, asthenia, somnolence, dizziness, tremor, weight gain, and alopecia. Because most side effects are dose related, patient and physician should aim for the lowest possible therapeutic dose.

Valproate has a known risk of hepatic failure, particularly in young children. Liver function tests need to be performed at regular intervals. Valproate has been reported to produce teratogenic effects such as neural tube defects.

Nevertheless, the prevention of a migraine attack is preferable over suppression of an attack, because prophylactic treatment allows the patient greater freedom from the disease. This is especially true in more severe cases where patients have a higher frequency of attacks. The ultimate goal in all cases is complete freedom from any further attack, managed through continuing prophylactic treatment. Until now, such a goal has only been achieved with valproate, but at a serious price of side effects as mentioned above and contraindications (e.g. interactions with other medications and particularly potential for congenital malformations).

There is a genuine need to develop other alternatives and to provide a compound with a therapeutic margin that is more appropriate to the treatment and more particularly for the prophylactic treatment of this pathology.

*Major depressive disorder (MDD)* as used herein is a common disorder of mood and affect characterized by one or more major depressive episodes. These episodes are defined diagnostically using a criteria-based syndrome listed and described in the Diagnostic and Statistical Manual Series, 4th Ed. (DSM-IV) (American Psychiatric Association Press, Washington, D.C., 1994). These episodes are diagnosed in a human patient if the patient has experienced five symptoms from a list of nine symptom categories every day, or nearly every day, for a period lasting at least two weeks. At least one symptom must be present from either category 1 (having a sad, depressed, empty or irritable mood or appearing sad to others) or category 2 (experiencing loss of interest in or pleasure from activities). The other symptom categories include: 3) change in weight and/or appetite, 4) insomnia or hypersomnia, 5) psychomotor agitation or retardation, 6) fatigue and/or loss of energy, 7) feelings of worthlessness and/or excessive or inappropriate guilt, 8) diminished ability to think or of concentrate and/or indecisiveness, and 9) recurrent thoughts of death or suicide.

The diagnostic terms and codes from DSM-IV are Major depressive disorder, single episode (DSM IV-296.2) and Major depressive disorder, recurrent (DSM IV-296.3).

A variety of pharmacologic agents are available for the treatment of depression. Significant success has been achieved through the use of serotonin reuptake inhibitors (SRIs), norepinephrine reuptake inhibitors (NERIs), combined serotonin-norepinephrine reuptake inhibitors (SNRIs), monoamine oxidase inhibitors (MAOIs), phosphodiesterase-4 (PDE4) inhibitors or other compounds. However, even with these options available, many patients fail to respond, or respond only partially to treatment. Additionally, many of these agents show delayed onset of activity, so that patients are required to undergo treatment for weeks or months before receiving benefits. Most currently available antidepressants take 2-3 weeks or more to elicit a response.

Traditional therapies can also have significant side effects. For example, more than a third of patients taking SRIs experience sexual dysfunction. Other problematic side effects include gastrointestinal disturbances, often manifested as nausea and occasional vomiting, agitation, insomnia, weight gain, onset of diabetes, prolongation of the heart rate corrected interval (QTc), agranylocytosis, etc. Depressive patients who also suffer from psychotic disorders (e.g. schizophrenia) also sometimes suffer extrapyramidal side effects. These side effects often discourage patients from following their recommended therapeutic regimen.

There remains a need for the development of improved therapies for the treatment of depression and/or other mood disorders.

Depression is common in people with epilepsy. A number of anticonvulsants are implicated in causing depression. All classes of antidepressants are reportedly successful in treating epilepsy-related depression but may also, to a varying degree, lower seizure control and thus pose a difficulty in treating depression in epilepsy (Lowe, A, The Impact of Depression. www.epilepsy.ca). The anticonvulsant levetiracetam has been reported to induce depression, and common pathogenic mechanisms for depression and epilepsy suggested (Wier, L M et al., Depression in Epilepsy. A New Look at This Disorder. J clin psychiatry (2006) 67(7):1159-1160).

*Fibromyalgia* as used herein is a chronic syndrome characterized by diffuse or specific muscle, joint and/or bone pain, fatigue and a wide range of other symptoms. Recent studies suggest that people with fibromyalgia may be genetically predisposed. It affects more females than males, with a ratio of 9:1 by ACR (American College of Rheumatology) criteria. Fibromyalgia is seen in 3- 6 % of the general population, and is most commonly diagnosed in individuals between the ages of 20 and 50. The nature of fibromyalgia is not well understood, with many frustrated physicians driven to accusing their patients of feigning illness. There are few, if any, treatments available. Although there is no cure, the disease itself is neither life-threatening nor progressive, though the degree of symptoms may vary greatly from day to day with periods of flares (severe worsening of symptoms) or remission.

The primary symptom of fibromyalgia is widespread, diffuse pain, often including heightened sensitivity of the skin, tingling of the skin (often needlelike), achiness in the muscle tissues, prolonged muscle spasms, weakness in the limbs, and nerve pain. Chronic sleep disturbances are also characteristic of fibromyalgia; it is not unusual for patients to experience extended periods (two hours or more) of sleep inertia.

Other symptoms often attributed to fibromyalgia are physical fatigue, irritable bowel syndrome, genitourinary symptoms such as those associated with the chronic bladder condition, interstitial cystitis, dermatological disorders, headaches, myoclonic twitches and symptomatic hypoglycemia. Although it is common in people with fibromyalgia for pain to be widespread, it may also be localized in areas such as the shoulders, neck, back, hips or other areas.

Fibromyalgia can start as a result of a trauma (such as a traffic accident) or major surgery, but there is currently no known strong correlation between any specific type of trigger and the subsequent initiation of fibromyalgia. Symptoms can have a slow onset, and many patients have mild symptoms beginning in childhood, such as growing pains. Symptoms are often aggravated by unrelated illness or changes in the weather. They can become more tolerable or less tolerable throughout daily or yearly cycles; however, many people with fibromyalgia find that, at least some of the time, the condition prevents them from performing normal activities such as driving a car or walking up stairs. The syndrome does not cause inflammation as is presented in arthritis, but anti-inflammatory treatments, such as ibuprofen is known to temporarily reduce pain symptoms in some people.

As with many other soft tissue and rheumatolgical organic disorders, there is no cure for fibromyalgia. However, there is increasing interest and research which has led to improvements in treatment. Treatment ranges from symptomatic prescription medication to alternative and complementary medicine. Low doses of antidepressants may be used to reduce the sleep disturbances sometimes associated with fibromyalgia and are believed by some practitioners to help correct sleep problems that may exacerbate the symptoms of the condition.

New drugs claiming efficacy on fibromyalgia pain and other symptoms include milnacipran, gabapentin, meloxicam and pregabalin but none of them represents any breakthrough in the treatment of fibromyalgia

*Bipolar disorder* as used herein is defined as Mood Disorder *(*Diagnostic and Statistical Manual of Mental Disorders (DSM-IV TM), 4th Ed., American Psychiatry Association, Washington, D.C., 1994). Bipolar disorder is generally characterized by spontaneously triggered repeated (i.e. at least two) episodes in which the patient's hyperexcitability, activity and mood are significantly disturbed, this disturbance consisting on some occasions of an elevation of mood and increased energy and activity (mania or hypomania), and in other occasions a lowering of mood and decreased energy and activity (depression).

Bipolar disorders are separated into four main categories: bipolar I disorder, bipolar II disorder, cyclothymia, and bipolar disorders not otherwise specified.

The essential feature of bipolar I disorder is a clinical course that is characterized by one or more manic episodes alternated with one or more major depressive episodes.

The essential feature of bipolar II disorders is a clinical course that is characterized by one or more major depressive episodes accompanied by at least one hypomanic episode. No full manic or mixed episodes are present.

*Cyclothymia* as used herein is a condition characterized by numerous periods of hypomanic symptoms that do not meet criteria for a manic episode and periods of depressive symptoms that do not meet symptom or duration criteria for a major depressive episode.

Bipolar disorder not otherwise specified may be diagnosed in addition to the diagnosis of schizophrenia, delusional disorders, or psychotic disorder not otherwise specified. If there is a very rapid alternation (over days) between manic symptoms and depressive symptoms (e.g. several days of purely manic symptoms followed by several days of purely depressive symptoms) that do not meet minimal duration criteria for a manic episode or major depressive episode, the diagnosis is bipolar disorders not otherwise specified.

*Manic episode* as used herein is a distinct period of time during which there is an abnormally and persistently elevated, expansive and/or irritable mood with signs of pressured speech and psychomotor agitation.

*Hypomania* as used herein is a less extreme or minor manic episode, with a lower grade of severity.

*Major depressive episode* as used herein is a period of at least two weeks during which there is either depressed mood and/or loss of interest or pleasure in nearly all activities with signs of impaired concentration and psychomotor retardation.

*Mixed episode* as used herein is a period of time (lasting at least one week) in which the criteria are met both for a manic episode and for a major depressive episode nearly every day.

*Depression relapse* as used herein is an episode of major depressive disorder occurring within six months from either response or remission.

For a number of decades, the treatment of mania and manic recurrences in bipolar disorders has essentially been based on the use of lithium salts, e.g. lithium sulphate, lithium carbonate and lithium citrate. In recent years, the incomplete protection and tolerance furnished by long-term use of Li⁺ for bipolar disorders has led to alternative treatments being considered. Clinical studies indicate that during the acute phase of bipolar disorder, up to 40 % of patients do not satisfactorily respond to lithium treatment (Gustavo A. et al., Anticonvulsants for treatment of manic depression. Current Drug Therapy (1989) 56(8)).

A number of safety problems linked to long-term use of lithium salts have been observed. Thus chronic interstitial nephropathy, polyuria, diabetes insipidus or nephrogenic diabetes insipidus occur in 25 % of subjects treated over a period of more than two years. Further, normal use of lithium salts frequently induces dysarthria, trembling, ataxia, hypothyroidism (30 % of subjects in the first two years) and impotence.

One of the most common alternative treatments is the use of valproate, which has been shown to have an anti-manic activity and is also capable of having a mood stabilizing activity. However, the results obtained are not yet satisfactory, and moreover valproate readily induces a variety of side effects. The usual side effects of valproate relate to the gastrointestinal tract, such as nausea, vomiting, anorexia and diarrhoea, as described in the preceding paragraph.

There is a genuine need to develop other alternatives to lithium salts and valproate to avoid their numerous side effects and to provide a compound with a therapeutic margin, which is more appropriate to the treatment of this pathology. *Chronic pain* as used herein is a condition distinct from acute pain. Conventionally defined as pain that persists beyond the normal time of healing, chronic pain can also be considered chronic at the point when the individual realises that the pain is going to be a persistent for the foreseeable future. It is likely that a majority of chronic pain syndromes involve a neuropathic component, which is usually harder to treat than acute somatic pain.

The best examples of predominantly neuropathic pain syndromes are diabetic peripheral neuropathy and post herpetic neuralgia. Pain is also many times present in diseases such as diabetes, HIV and hepatitis. A primarily somatic chronic pain syndrome is exemplified by patients with rheumatoid arthritis or other rheumatologic diseases. On the other hand, the most common chronic pain syndrome involving back injury related pain often involves multiple organ systems. The other major cause of chronic pain is cancer, which is known for its ability to cross tissue boundaries and damage or compress a variety of organ systems. Therefore, most back injury and cancer patients have pain related to both somatic and neuropathic mechanisms (Hansen H C. "Treatment of chronic Pain With Antiepileptic Drugs: A New Era" South Medical Journal-Southern Medical Association (1999) 92(7):642-649).

*Neuropathic pain* as used herein is pain initiated by a pathological change in a nerve, which signals the presence of a noxious stimulus when no such recognisable stimulus exists, giving rise to a false sensation of pain. In other words, it appears that the pain system has been turned on and cannot turn itself off. Neuropathic pain may be related to peripheral or central (spinal or brain) nerve lesions or dysfunction in the nervous system. Neuropathic pain can be manifest as a result of conditions such as nerve injury (e.g. surgery, accident, amputation), trauma affecting the limb (with or without obvious nerve lesions), diseases affecting the nervous system, infarct related to the nervous system, abnormal nerve function, spinal and radicular pain disorders.

*Nerve injury* as used herein is a condition giving rise to a pain sensation such as phantom pain (pain referred to the amputated limb), stump pain (pain at the amputation site), phantom limb (non-painful sensations referred to the amputated limb), post-operative pain, thalamic pain syndrome (central post-stroke pain). By *trauma affecting a limb* as used herein is meant a condition selected from reflex symptomatic dystrophy, causalgia. By *diseases affecting the nervous system* as used herein is meant a condition selected from diabetic neuropathy and other neuropathies, trigeminal neuralgia (TN), post-herpetic neuralgia (PHN), multiple sclerosis, AIDS-related neuropathy, cancer-related neuropathy (neuropathy secondary to chemotherapy) (Troel S and Jensen M D, Mechanisms of Neuropathic Pain. Pain (1996) 77-86).

Chronic and/or neuropathic pains remain the pain syndromes that are the most difficult to treat and there is a genuine need to develop novel active ingredients.

For almost thirty years, very little progress has been made in the drug treatment of chronic pain and neuropathic pain and this remains restricted to the use of antidepressants, non-steroidal anti-inflammatory drugs (NSAIDs), local anaesthetics and anticonvulsants.

A number of anticonvulsants, such as valproate and carbamazepine, possess activity in the treatment of these pain conditions, but others such as pentobarbital are ineffective (Fields H L et al., Excitability Blockers: anticonvulsants and low concentration local antethetics in the treatment of chronic pain, in: Dickenson A J and Besson J-M (eds). Handbook of Experimental Pharmacology vol. 130, 93-116. The Pharmacology of Pain. Springer Verlag, Berlin 1997; Hansen H C., *supra).*

Carbamazepine, an antiepileptic drug, acts as a reference for pharmacological studies in the field of chronic or neuropathic pain. It has a distinct activity in inhibiting artificially induced pain or hyperalgesia. However, pharmacological studies have confirmed that the dose-activity curve does not only reverse the pain to the control threshold (reversal of hyperalgesia), but it goes beyond that and induces partial desensitization in treated subjects. Thus with sensitivity threshold being higher than normal, the patient would be much less receptive to any external aggressions such as heat, chafing or the like, and thus would risk injuring or burning him- or herself.

Considerable precautions must be taken when using carbamazepine, which is a first line treatment, since the difference between the therapeutic dose and the dose inducing side effects, is extremely small. Further, the posology inducing these effects varies depending on the patient. Thus the practitioner must be extremely vigilant when adjusting the doses to each individual treated. Side effects can include sedation, ataxia, dizziness, blurred vision, also nausea and vomiting. Furthermore, about 10 % of patients exhibit mild leucopoenia (Fields H L et al., Excitability Blockers 93-116).

Russian patent no. 2144918 discloses the anticonvulsant 3-[(benzylamino)-methylidene]-pyrrolidine-2,4-dione, a tetramic acid derivative; an improved method for its preparation is described in Russian patent no. 2223266.

Derivatives of 3-ethylidenepyrrolidine-2,4-dione disclosed in Japanese patent no. 49020332 have been found effective against fungal and bacterial microorganisms. Similar pyrrolidinediones are disclosed in Japanese patent no. 48034741 as anticonvulsants and tranquilizers. Other pyrrolidinediones are disclosed in Japanese patents nos. 48028465 and 52010266 as anti-cancer and herbicidal agents, respectively. 5-Substituted 3-(1'-anilinoethylidene)pyrrolidine-2,4-diones are disclosed as anti-tumour agents (Yuki, H et al., Gann 62;3 (1971) 199-206).

### OBJECTS OF THE INVENTION

It is an object of the invention to provide a compound for use in a method of curative or prophylactic treatment of a person suffering from a peripheral or central nervous system disorder or being at risk of developing such a disorder.

Additional objects of the invention will become evident from the following summary of the invention, the description of preferred embodiments thereof, and the appended claims.

### SUMMARY OF THE INVENTION

According to the present invention is provide a compound for use in a method of curative or prophylactic treatment of a person suffering from a peripheral or central nervous system disorder or condition or being at risk of developing such a disorder or condition, the compound being 3-phenylmethyl-amino-methylene)pyrrolidine-2,4-dione (I) 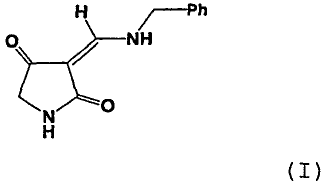 or a pharmaceutically acceptable salt, or solvate thereof. The compound of the invention as prepared by the method disclosed in Russian Patent No. 2144918 is an about 1:1 mixture of cis/trans isomers in respect of the carbon-carbon double bond. The present invention comprises 3-phenylmethyl-amino-methylene)pyrrolidine-2,4-dione in any cis/trans isomer ratio as well in form of the pure cis and trans isomers.

According to the invention the peripheral or central nervous system disorder or condition is selected from neuropathic pain, chronic pain, migraine, fibromyalgia, bipolar disorder and depressive disorder.

Preferred embodiments of the invention are disclosed in the appended claims 2 to 9.

Administration of 3-phenylmethyl-amino-methylene)pyrrolidine-2,4-dione (I) or a pharmaceutically acceptable salt, prodrug or solvate thereof in the method of the invention is preferably for gastrointestinal absorption, in particular by the oral or rectal route, such as in form of a tablet, capsule, lozenge, suppository, and the like. It is also within the scope of the invention to provide liquid formulations, in particular aqueous formulations, for oral administration. A further preferred route of administration is by intravenous or intra-muscular injection in a liquid carrier such as physiological saline.

A daily dosage of 3-phenylmethyl-amino-methylene)pyrrolidine-2,4-dione (I) or a pharmaceutically acceptable salt, prodrug or solvate thereof administered to the patient can fall within a wide range of concentrations and depends on a variety of factors such as the patient's sex, age, weight and medical condition, as well as on the method of administration. It is best determined by the attending doctor. A preferred daily dose of 3-phenylmethyl-amino-methylene)pyrrolidine-2,4-dione (I) or a corresponding molar amount of a pharmaceutically acceptable salt, prodrug or solvate thereof for an adult person is from 200 mg to 800 mg. The quantity of active ingredient in compositions for oral administration is at least 0.5 % by weight and can be up to 80 % by weight with respect to the composition weight. A suitable tablet for oral administration comprising about 50 per cent by weight of 3-phenylmethyl-amino-methylene)pyrrolidine-2,4-dione (I) is disclosed in Russian Patent No. 2144917, which is incorporated herein by reference. Other suitable capsule and tablet compositions for oral administration are disclosed in Russian Patent No. 2223216, which is incorporated herein by reference.

The invention will now be explained in greater detail by reference to preferred but not limiting embodiments.

### DESCRIPTION OF PREFERRED EMBODIMENTS OF THE INVENTION

The therapeutically efficiency of the compounds of the invention in regard of the conditions to which it relates is supported by the following examples.

### Analgesic effect

### EXAMPLE 1

*Hot plate* test. Male mice weighing from 25 to 30 g were used in this test. The thermal technique (hot plate analgesia meter) was used to evaluate analgesic effect after the injection of drugs or saline. The surface of the plate was maintained at a temperature of 55.0 ± 0.5 °C. Analgesic effect was determined 30 min. after compound injection. Time (in seconds) to either hind paw licking or jumping was recorded as pain response latency. To avoid the likelihood of tolerance of mice to hot plate, all mice were exposed to hot plate only once through the whole experiment. The mice were exposed to hot plate only once; either before or after injection of drug.

In this hot plate test the tetramic derivative of the invention exhibits an analgesic effect after peroral administration in mice at a dose of 50 mg/kg. A similar effect was observed with phenytoin and valproate at 100 and 500 mg/kg respectively.

### EXAMPLE 2

*Tail-flick test.* The tail-flick test, using a radiant heat source was carried out to study antinociception. Each mouse was placed in an acrylic tube and its tail was laid across a Nichrome™ wire coil, which was heated by the passage of an electric current. The intensity of the current was kept at 5 mA. All animals were screened for the tail-flick response. Only those showing a flick within 4 sec were included in the study. After 1 h of final dosing, the mice were submitted to three tail-flick tests.

In this tail-flick test the tetramic derivative of the invention exhibits an analgesic effect after peroral administration.

At the highest doses tested (500 mg/kg), the tetramic derivative of the invention exhibits a relatively better analgesic effect compared to phenytoin and valproate (100 and 500 mg/kg respectively). All data were compared with the same control group.

### EXAMPLE 3

*Formalin paw test*. The mice received an injection of formalin (4.5 %, 20 µl) to the left hind paw. The irritation caused by the formalin injection elicits a characteristic biphasic behavioral response as quantified by the amount of time spent licking the injured paw. The first phase (∼5-10 minutes) represents direct chemical irrigation, and the second phase (∼20-30 minutes) is thought to represent pain of neuropathic origin (Tjølsen A et al. Pain (1992) 51:5-17). The two phases are separated by a quiescent period in which behavior returns to normal. The effectiveness of test compounds to reduce the painful stimuli is assessed by counting the amount of time spent licking the injured paw in the two phases.

In this inhibition of pain behavior test, the tetramic derivative of the invention displays a 5 % reduction in pain score in the chronic phase at a dose of 30 mg/kg. Similar reduction was seen with gabapentin and morphine at 20 mg/kg and 1.25 mg/kg, respectively, compared to control.

### Depression

### EXAMPLE 4

*Forced Swim Test.* The test apparatus consisted of a clear plastic cylindrical tank, 35 cm high and 25 cm in diameter, which was filled to a depth of 25 cm with tap water equilibrated overnight to room temperature (24 °C). A glass plate covered the top of the tank to prevent escape while the mouse was swimming. The water in the tank was replaced by fresh water at room temperature after three mice had been tested. To habituate the mouse, it was forced to swim in the tank for 15 min., then removed and dried by toweling and a heat lamp, and returned to its cage. Twenty-four hours later, the mouse was returned to the tank and allowed to swim for 5 min. while its swimming activity was recorded by a video camera, mounted above the tank. After this, the mouse was dried and returned to its cage. End points of the test were total time spent floating in an immobile position, without paddling of limbs (swimming) (Porsolt et al. Nature (1977) 266:730-732).

In this test, a significant antidepressive effect of the tetramic derivative of the invention was observed after administration of 100 mg/kg. Phenobarbital, carbamazepine and sodium valproate showed significant effect after administration of 25, 50 and 250 mg/kg, respectively. Animals in each experimental group served as their own control.

## Claims

1. 3-(Phenylmethyl-aminomethylene)-pyrrolidine-2,4-dione (I) 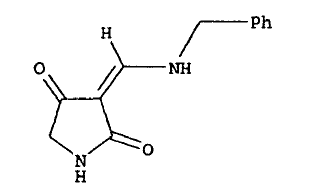 or a pharmaceutically acceptable salt or solvate thereof for use in a method of curative or prophylactic treatment of a person suffering from a peripheral or central nervous system disorder or condition or being at risk of developing such a disorder or condition, wherein the peripheral or central nervous system disorder or condition is selected from neuropathic pain, chronic pain, migraine, fibromyalgia, bipolar disorder and depressive disorder.

2. The compound of claim 1, wherein neuropathic pain is selected from pain related to diabetic neuropathy, pain related to HIV disease, pain related to hepatitis and pain related to post herpetic neuropathy.

3. The compound of claim 1, wherein chronic pain is selected from post-operative pain, cancer pain and resistant chronic pain.

4. The compound of claim 1, wherein migraine is selected from migraine without aura, migraine with aura, ophtalmoplegic migraine, retinal migraine, childhood migraine, status migrainous and migrainous infarction.

5. The compound of claim 1, wherein said central nervous system disorder is a bipolar disorder, and is bipolar I disorder selected from the group consisting of bipolar disorder accompanied by a recent hypomanic, manic, mixed, depressed and unspecific episode, or bipolar II disorder selected from bipolar disorder with recurrent major depressive episodes including hypomanic episodes and cyclothymic disorder.

6. The compound of claim 1, wherein said bipolar disorder is bipolar depression or treatment resistant bipolar disorder.

7. The compound of claim 1, wherein said depressive disorder is selected from major depressive disorder, depression relapse, recurrent depression and resistant depression.

8. The compound of claim 1, wherein said condition or disorder is migraine.

9. The compound of claim 1, wherein said condition or disorder is fibromyalgia.

## Patentansprüche

1. 3-(Phenylmethyl-Aminomethylen)-Pyrrolidin-2,4-Dion (I) 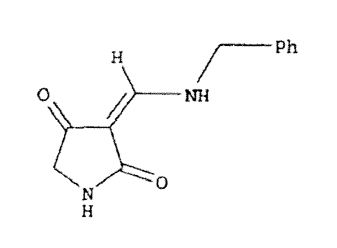 oder ein pharmazeutisch zulässiges Salz oder Solvat davon zur Verwendung in einem Verfahren zur Heil- oder prophylaktischen Behandlung einer Person, die an einer Erkrankung oder an einem Zustand des peripheren oder zentralen Nervensystems oder an einem Risiko, so eine Erkrankung oder so einen Zustand zu entwickeln, leidet, wobei die Erkrankung oder der Zustand des peripheren oder zentralen Nervensystems aus neuropathischem Schmerz, chronischem Schmerz, Migräne, Fibromyalgie, bipolarer Störung und depressiver Störung ausgewählt ist.

2. Verbindung nach Anspruch 1, wobei der neuropathische Schmerz aus diabetische Neuropathie betreffendem Schmerz, HIV-Erkrankung betreffendem Schmerz, Hepatitis betreffendem Schmerz und postherpetische Neuropathie betreffendem Schmerz ausgewählt ist.

3. Verbindung nach Anspruch 1, wobei der chronische Schmerz aus postoperativem Schmerz, Krebsschmerz und beständigem chronischem Schmerz ausgewählt ist.

4. Verbindung nach Anspruch 1, wobei die Migräne aus Migräne ohne Aura, Migräne mit Aura, ophtalmopiegischer Migräne, retinaler Migräne, Kindheitsmigräne, Status migränosus und migränösem Infarkt ausgewählt ist.

5. Verbindung nach Anspruch 1, wobei die Erkrankung des zentralen Nervensystems eine bipolare Störung ist und eine bipolare-I-Störung ist, die aus der aus bipolaren Störungen, die von einer kürzlich hypomanischen, manischen, gemischten, depressiven und unspezifischen Episode begleitet waren, bestehenden Gruppe ausgewählt ist, oder eine bipolare-II-Störung ist, die aus bipolaren Störungen mit wiederkehrenden größeren depressiven Episoden einschließlich hypomanischer Episoden und aus cyclothymischen Störungen ausgewählt ist.

6. Verbindung nach Anspruch 1, wobei die bipolare Störung eine bipolare Depression oder Behandlung einer resistenten bipolaren Störung ist.

7. Verbindung nach Anspruch 1, wobei die depressive Störung aus größerer depressiver Störung, Depressionsrückfall, wiederkehrender Depression und beständiger Depression ausgewählt ist.

8. Verbindung nach Anspruch 1, wobei der Zustand oder die Erkrankung Migräne ist.

9. Verbindung nach Anspruch 2, wobei der Zustand oder die Erkrankung Fibromyalgie ist.

## Revendications

1. 3-(Phénylméthyl-aminométhylène)-pyrrolidine-2,4-dione (I) 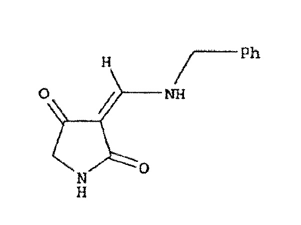 ou un sel pharmaceutiquement acceptable ou un solvate de celle-ci pour une utilisation dans un procédé de traitement curatif ou prophylactique d'une personne souffrant d'un trouble ou d'une affection du système nerveux périphérique ou central ou étant à risque de développer un tel trouble ou une telle affection, où le trouble ou l'affection du système nerveux périphérique ou central est choisi parmi la douleur neuropathique, la douleur chronique, la migraine, la fibromyalgie, le trouble bipolaire et le trouble dépressif.

2. Composé selon la revendication 1, où la douleur neuropathique est choisie parmi la douleur associée à une neuropathie diabétique, la douleur associée à la maladie du VIH, la douleur associée à une hépatite et la douleur associée à une neuropathie postherpétique.

3. Composé selon la revendication 1, où la douleur chronique est choisie parmi la douleur postopératoire, la douleur due à un cancer et la douleur chronique résistante.

4. Composé selon la revendication 1, où la migraine est choisie parmi la migraine sans aura, la migraine avec aura, la migraine ophtalmoplégique, la migraine rétinienne, la migraine de l'enfant, l'état migraineux et l'infarctus migraineux.

5. Composé selon la revendication 1, où ledit trouble du système nerveux central est un trouble bipolaire, et est un trouble bipolaire I choisi dans le groupe constitué du trouble bipolaire accompagné d'un épisode récent hypomaniaque, maniaque, mixte, déprimé ou non spécifique, ou un trouble bipolaire II choisi parmi un trouble bipolaire avec des épisodes dépressifs majeurs récurrents y compris des épisodes hypomaniaques et un trouble cyclothymique.

6. Composé selon la revendication 1, où ledit trouble bipolaire est une dépression bipolaire ou un trouble bipolaire résistant aux traitements.

7. Composé selon la revendication 1, où ledit trouble dépressif est choisi parmi le trouble dépressif majeur, la rechute de dépression, la dépression récurrente et la dépression résistante.

8. Composé selon la revendication 1, où ladite affection ou ledit trouble est la migraine.

9. Composé selon la revendication 1, où ladite affection ou ledit trouble est la fibromyalgie.
